(19) Europäisches Patentamt
European Patent Office
Office européen des brevets

(11) EP 4 219 001 A1

(12) EUROPEAN PATENT APPLICATION
published in accordance with Art. 153(4) EPC

(43) Date of publication:
02.08.2023 Bulletin 2023/31

(51) International Patent Classification (IPC):
B01J 20/18 (2006.01)        B01J 20/30 (2006.01)
C07C 7/13 (2006.01)

(21) Application number: 21871628.0

(22) Date of filing: 26.09.2021

(52) Cooperative Patent Classification (CPC):
B01J 20/18; B01J 20/30; C07C 7/13

(86) International application number:
PCT/CN2021/120556

(87) International publication number:
WO 2022/063259 (31.03.2022 Gazette 2022/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.09.2020 CN 202011044618

(71) Applicants:
• China Petroleum & Chemical Corporation
Beijing 100728 (CN)
• Shanghai Research Institute of Petrochemical
Technology, SINOPEC
Shanghai 201208 (CN)

(72) Inventors:
• JIA, Yinjuan
Shanghai 201208 (CN)

• GAO, Huanxin
Shanghai 201208 (CN)
• WU, Shuang
Shanghai 201208 (CN)
• WANG, Can
Shanghai 201208 (CN)
• YANG, Heqin
Shanghai 201208 (CN)
• JING, Mengmeng
Shanghai 201208 (CN)
• LIU, Zhicheng
Shanghai 201208 (CN)
• HE, Limin
Shanghai 201208 (CN)

(74) Representative: karo IP
karo IP Patentanwälte
Kahlhöfer Rößler Kreuels PartG mbB
Postfach 32 01 02
40416 Düsseldorf (DE)

(54) **ADSORBENT COMPOSITION, AND PREPARATION METHOD THEREFOR AND APPLICATION THEREOF**

(57) The present disclosure relates to an adsorbent composition, and a preparation method therefor and an application thereof. The adsorbent composition in accordance with the present disclosure comprises molecular sieves, hydrated alumina and alumina. The adsorbent composition in accordance with the present disclosure has a considerable adsorption capacity, is easy to regenerate, has excellent compressive strength, and does not have substantial adsorption heat release, and thereby is particularly suitable for removing polar compounds from low-carbon olefins.

Fig. 1

EP 4 219 001 A1

## Description

Field of the invention

The present disclosure relates to adsorption technology. In particular, the present disclosure relates to an adsorbent composition for removing polar compounds from low-carbon olefins, and its preparation method and application.

Background of the invention

Low-carbon olefins such as ethylene, propylene and butene are important industrial raw materials. They are widely used in petrochemical industry. For example, they can be polymerized to produce polyolefins, epoxidized to produce propylene oxide, butylene oxide, and the like, or alkylated with aromatics to produce ethylbenzene, cumene, diisopropylbenzene, and the like. Industrial low-carbon olefins usually contain polar compounds such as $H_2O$, methanol, ammonia, $H_2S$ and the like as impurities. The presence of such polar compounds may adversely affect the polymerization catalyst, epoxidation catalyst and alkylation catalyst used in the subsequent procedures, affecting the efficiency and life of the catalysts. Therefore, the efficient removal of polar compounds is significant for protecting the catalysts in the downstream devices and maintaining the long-term stable operation of the devices.

Adsorption method is widely used for the removal of polar compounds due to its advantages such as simple operation, low energy consumption, and the like. Bentonite, clay, kaolin, molecular sieve or the like is generally used as the adsorbent. Generally, the adsorbent is placed upstream of the reaction vessel containing catalysts to remove the polar compounds from the low-carbon olefin feedstocks and thereby reduce the catalyst poisoning.

CN1461290A discloses a process for removing polar impurities from an aromatic feedstock. The process comprises: contacting the aromatic feedstock containing polar compounds with an adsorbent, wherein said adsorbent comprises a molecular sieve having pores and/or surface cavities with cross-sectional dimensions greater than 5.6 Angstroms; and feeding the resultant treated aromatic feedstock to an alkylation reaction unit.

CN103418164B discloses a method for removing oxygen-containing compounds in a hydrocarbon stream by using a porous metal organic compound as a solid absorption agent to remove the oxygen-containing compounds from the hydrocarbon stream. The solid absorption agent is a porous metal organic compound of the formula of $M_3(BTC)_2(L)_3m$, wherein M is at least one transition metal element selected from the group consisting of Cu, Co, Fe, Ni, Zn and Cr, BTC represents deprotonated pyromellitic acid, L is at least one solvent molecule selected from the group consisting of $H_2O$, $NH_3$, $CH_3OH$, DMF, THF and $C_2H_5OH$, m represents the average number of solvent molecules combined with each metal ion, and $0 \leq m \leq 1$.

CN107970781A discloses a molecular sieve ceramic membrane material for olefin purification as well as preparation method and application of the same. In the molecular sieve ceramic membrane material, the particle sizes of molecular sieve particles supported on the surface of the ceramic material are 0.1-3 $\mu$m, and the thickness of the molecular sieve layer is 3-5 $\mu$m. During preparation, ceramic material pretreatment, molecular sieve seed crystal precoating and sealed crystallization are sequentially carried out to obtain the molecular sieve ceramic membrane material. The molecular sieve ceramic membrane material is used for removing polar oxygen-containing compounds in a gaseous olefin flow to a level of 1ppm or below.

However, there are still problems in the existing processes for removing polar compounds, for example, low adsorption capacity of the adsorbent, non-regeneration of the adsorbent, large discharge of solid waste, substantial adsorption heat release, and easy to cause olefin polymerization. Olefins are unsaturated hydrocarbons and have strong polarity. Therefore, when using molecular sieves to remove polar compounds from low-carbon olefins, the low-carbon olefins may be adsorbed while polar compounds are adsorbed, resulting in a sharp increase of adsorption heat, which in turn may cause the low-carbon olefins to be polymerized on the surface of the adsorbent. At the same time, molecular sieves are difficult to desorb after adsorbing polar compounds, resulting in incomplete regeneration. In addition, molecular sieve has limited strength, which limits its flexibility of application.

Accordingly, there is still a demand for further modification of molecular sieves and development of new adsorbent compositions, so as to remove polar compounds more effectively.

Summary of the invention

In order to solve one or more of the above problems, provided in this disclosure is an adsorbent composition, which comprises molecular sieves, hydrated alumina and alumina. The adsorbent composition in accordance with the present disclosure has a considerable adsorption capacity, is easy to regenerate, has excellent compressive strength, and does not have substantial adsorption heat release, and thereby is particularly suitable for removing polar compounds from low-carbon olefins. The present disclosure also relates to the preparation method and application of the adsorbent composition.

In the first aspect of the present disclosure, provided is an adsorbent composition for removing polar compounds from low-carbon olefins, comprising molecular sieves, hydrated alumina and alumina. Preferably, the adsorbent composition comprises, by weight: a) 10-50 parts, preferably 20-40 parts of the molecular sieves; b) 20-55 parts, preferably 30-45 parts of the hydrated alumina, calculated as alumina; c) 5-35 parts, preferably 8-30 parts of the alumina. More preferably, the adsorbent composition has a total amount of strong acids of less than 0.05mmol/g, preferably 0.01-0.04mmol/g.

In a further aspect of the present disclosure, provided is a method for preparing an adsorbent composition for removing polar compounds from low-carbon olefins, comprising the steps of: subjecting molecular sieves, hydrated alumina and alumina to mixing, shaping, drying and calcining to obtain the adsorbent composition, wherein the calcining is operated at a temperature of lower than 400 °C.

In more further aspect of the present disclosure, provided is a use of the adsorbent composition in removing polar compounds from low-carbon olefins. Preferably, the low-carbon olefins comprise ethylene, propylene, butene and the like, and the polar compounds comprise $H_2O$, methanol, ammonia, $H_2S$, COS and the like.

The adsorbent composition in accordance with the present disclosure comprises the combination of molecular sieves, hydrated alumina and alumina, which may adjust the pore structure, acidity and polarity of the resultant adsorbent composition. Therefore, the adsorbent composition in accordance with the present disclosure can desorb polar compounds, such as ammonia, at a lower temperature, and is easy to be regenerated in situ. In addition, the adsorbent composition in accordance with the present disclosure can reduce and avoid local temperature rise during adsorption, thereby reducing and avoiding the risk of undesirable polymerization of olefins. Further, the adsorbent composition in accordance with the present disclosure has improved compressive strength and thereby better long-term stability. Therefore, the adsorbent composition in accordance with the present disclosure has a broader application prospect.

Brief description of the drawings

The accompanying drawings are included to provide a further understanding of the invention and are a part of this specification, which, together with the following detail description of the invention, illustrate the invention but not intend to limit the scope thereof. In the drawings,

Fig. 1 shows the breakthrough curve of the adsorbent composition prepared in Example 1 for adsorption of $NH_3$ and $H_2S$;
Fig. 2 shows the $NH_3$-TPD curve of the adsorbent composition prepared in Example 1 after one or more regenerations;
Fig. 3 shows the XRD pattern of the adsorbent composition prepared in Example 1.

Detailed description of the invention

It should be understood that the endpoints and any values in the ranges disclosed herein are not limited to the precise range or value, but to encompass values close to those ranges or values. For ranges of values, it is possible to combine between the endpoints of each of the ranges, between the endpoints of each of the ranges and the individual points, and between the individual points to give one or more new ranges of values as if these ranges of values are specifically disclosed herein.

Other than in the examples, all numerical values of parameters in this specification are to be understood as being modified in all instances by the term "about" whether or not "about" actually appears before the numerical value.

As used in the specification, the term "low-carbon olefins" refers to olefins with 2-4 carbon atoms, including ethylene, propylene, butene and the like.

As used in the specification, the term "acidity" refers to the amount of active sites in the adsorbent used to adsorb alkaline polar compounds. Generally, the adsorbent shows two $NH_3$ desorption peaks, corresponding to different acidic sites of the adsorbent. Accordingly, the acidity can be measured via the $NH_3$-TPD curve of the adsorbent. Specifically, the distribution of different acidic sites of the adsorbent can be obtained by fitting the area of each desorption peak. In this specification, the active sites corresponding to a temperature of less than 300 °C in the $NH_3$-TPD curve of the adsorbent are regarded as weak acids, and the active sites corresponding to a temperature of greater than 300 °C are regarded as strong acids. The area of desorption peak of the corresponding strong acids or weak acids is obtained by fitting the desorption curve in the obtained $NH_3$-TPD profile of the adsorbent, and thereby the ratio of weak acid or strong acid to total acids is calculated. The total amount of acids can be measured by weighting via a microbalance. The total amount of strong acids or weak acids can be calculated based on the total amount of acids and the above ratios.

As used in the specification, the term "molecular sieve" is interchangeable with "zeolite" and "zeolite molecular sieve".

In one aspect, the present disclosure relates to an adsorbent composition for removing polar compounds from low-carbon olefins, which comprises molecular sieves, hydrated alumina and alumina. In one variant, the adsorbent composition comprises, by weight: a) 10-50 parts, preferably 20-40 parts of the molecular sieves; b) 20-55 parts, preferably 30-45 parts of the hydrated alumina, calculated as alumina; c) 5-35 parts, preferably 8-30 parts of the alumina.

In one variant, the adsorbent composition has a total amount of strong acids of less than 0.05mmol/g, preferably 0.01-0.04mmol/g.

In one variant, the XRD pattern of the adsorbent composition involves diffraction peaks at $2\theta$ of 6.03°, 13.47°, 15.32°, 28.01°, 37.96° and 49.28°, preferably involves diffraction peaks at $2\theta$ of 6.03°, 13.47°, 15.32°, 28.01°, 23.16°, 37.96°, 46.34°, 49.28° and 66.8°, more preferably involves diffraction peaks at $2\theta$ as outlined in the following table:

| $2\theta$ |
| --- |
| 6.03 |
| 9.87 |
| 11.6 |
| 13.47 |
| 15.32 |
| 19.9 |
| 23.16 |
| 26.5 |
| 28.01 |
| 30.8 |
| 31.8 |
| 33.4 |
| 37.96 |
| 46.34 |
| 49.28 |
| 64.7 |
| 66.8 |

From the XRD pattern of the adsorbent composition, it is possible to identify the derivative peaks attributable to the molecular sieves, for example, the diffraction peaks at $2\theta$ of 6.03°, 9.87°, 11.6°, 15.32°, 23.16°, 26.5° and 30.8°, to identify the derivative peaks attributable to hydrated alumina, for example, the diffraction peaks at $2\theta$ of 13.47°, 28.01°, 37.96°, 49.28° and 64.7°, and to identify the derivative peaks attributable to alumina, for example, the diffraction peaks at $2\theta$ of 46.34° and 66.8°.

Molecular sieve (or zeolite) has a skeleton with such a basic structure wherein $SiO_4$ and $AlO_4$ tetrahedras form a three-dimensional network structure by combining them via the shared oxygen atoms. Such combining may lead to voids and channels in molecular size and with uniform pore size. The above structure of zeolite imparts functions such as screening molecules, adsorbing, exchanging ions and catalyzing. In one embodiment, preferred are microporous molecular sieves with a pore size of less than or equal to 2 nm. In one variant, the molecule sieve is one or two selected from the group consisting of X-type molecular sieve and Y-type molecular sieve, preferably one or two selected from the group consisting of 13X and NaY.

The hydrated alumina may be for example pseudo-boehmite, boehmite, gibbsite, bayerite, and the like, preferably pseudo-boehmite. The hydrated alumina is commercially available or can be prepared according to the known technology in the art. In one embodiment, the hydrated alumina has a specific surface area of 200-500m$^2$/g, preferably 300-400m$^2$/g, and a pore volume of 0.2-0.5 cm$^3$/g, preferably 0.3-0.4 cm$^3$/g. In one variant, the hydrated alumina has a pore size of 2.0-5.0nm, preferably 4.0-5.0nm. In one variant, the hydrated alumina has a total amount of weak acids of 0.20-0.50mmol/g.

The alumina may be in any crystal phase structure, such as $\alpha$-alumina, $\gamma$-alumina, $\delta$-alumina, $\eta$-alumina and

the like, preferably γ-alumina. The alumina is commercially available or can be prepared according to the known technology in the art. In one embodiment, the alumina has a specific surface area of 100-400m$^2$/g, preferably 200-300m$^2$/g, and a pore volume of 0.4-1.0cm$^3$/g, preferably 0.5-0.8cm$^3$/g. In one variant, the alumina has a pore size of 5.0-10.0nm, preferably 6.0-8.0nm. In one variant, the alumina has a total amount of weak acids of 0.01-0.05mmol/g.

In one aspect, the present disclosure relates to a method for preparing an adsorbent composition for removing polar compounds from low-carbon olefins, comprising the steps of:
subjecting molecular sieves, hydrated alumina and alumina to mixing, shaping, drying and calcining to obtain the adsorbent composition, wherein the calcining is operated at a temperature of lower than 400 °C.

In one embodiment, the drying is operated at a temperature of 50-150 °C for 3-15 hours. The calcining is operated at a temperature of 200-400 °C, preferably 250-350 °C for 2-10 hours, preferably 3-8 hours.

The shaping is preferably extrusion. It is possible to use any extruders commonly used in the art for the shaping. The shaping may result in regular shapes or irregular shapes. In one embodiment, the shaping is extruding into stripes.

In order to facilitate the mixing and shaping, peptizing agents and extrusion aids may be further added. Examples of suitable peptizing agents include, for example, nitric acid and citric acid. Examples of suitable extrusion aids include, for example, sesbania powder and methyl cellulose. The amounts of peptizing agents and extrusion aids may be those commonly used in the art.

In one embodiment, the hydrated alumina is pseudo-boehmite. In one variant, the pseudo-boehmite is prepared by coprecipitation reaction of an aqueous solution of water-soluble metaaluminates and a nitric acid solution. Water-soluble metaaluminates include sodium metaaluminate, potassium metaaluminate and the like. Preferably, a sodium metaaluminate solution of 0.5 mol/L-1.5 mol/L reacts with a nitric acid solution of 0.5 mol/L-1.5 mol/L at pH=6-8. The reaction may be operated at a temperature of 40-90 °C for 0.5-3 hours. Pseudo-boehmite is obtained as a reaction precipitate, which is then washed and dried for the use in preparing the adsorbent composition in accordance with the present disclosure.

In one embodiment, the alumina is prepared from pseudo-boehmite. Preferably, the preparation method comprises: mixing pseudo-boehmite with a solution of polyacrylic acid or with a solution of ammonium polyacrylate or sodium polyacrylate, drying and calcining to obtain the alumina. In one variant, the mixing comprises mixing pseudo-boehmite with the solution of polyacrylic acid in a mass ratio of 1:0.5-1.5, wherein the solution of polyacrylic acid has a mass concentration of 0.5-1.5%. In one variant, the drying is operated at a temperature of 80-150 °C for 3-15 hours. The calcining is operated at a temperature of 400-600 °C for 3-10 hours.

The adsorbent composition in accordance with the present disclosure can be used in removing polar compounds from low-carbon olefins. Low-carbon olefins that can be treated may comprise ethylene, propylene, butene and the like. The polar compounds comprise $H_2O$, methanol, ammonia, $H_2S$, COS, mercaptan and other organic compounds containing S or O.

Without being bound by any theory, it is believed that the adsorbent composition in accordance with the present disclosure comprises a combination of molecular sieves, hydrated alumina and alumina with different pore structures, acidities and polarities, which adjusts the pore structure, acidity and polarity of the resultant adsorbent composition. Therefore, the adsorbent composition in accordance with the present disclosure has considerable adsorption capacity, is easy to regenerate, and can reduce and avoid local temperature rise during adsorption. In addition, the adsorbent composition in accordance with the present disclosure unexpectedly has improved compressive strength.

Examples

The present invention will be further described with the following examples. The examples are intended to illustrate and not to limit the invention in any way.

Testing methods

1. Acidity: NH$_3$-TPD test was conducted with the temperature programmed desorption device PX200A from Tianjin Golden Eagle Technology Co., Ltd, at the conditions of ammonia adsorption temperature being 30 °C, the carrier gas being He, the flow rate being 30 mL/min, and the heating rate being 10 °C /min. The area of desorption peaks of corresponding strong acids or weak acids was obtained by fitting the desorption curve in the obtained NH$_3$-TPD profile, and thereby the ratio of weak acids or strong acids to total acids was calculated, wherein the acidic sites corresponding to the curve at a temperature of less than 300 °C were regarded as weak acids, and the acidic sites corresponding to the curve at a temperature of greater than 300 °C were regarded as strong acids. The total amount of acids was measured by weighting via a microbalance. The total amount of strong acids or weak acids was calculated based on the total amount of acids and the above ratios.

2. Specific surface area: the adsorption curve of the sample was obtained with ASAP2600 surface analyzer from US. Based on the adsorption curve, the specific surface area of the sample was calculated according to the BET method.

3. Pore volume: the adsorption curve of the sample was obtained with ASAP2600 surface analyzer from US. The total pore volume was calculated according to the single-point method.

4. Pore size: the adsorption curve of the sample was obtained with ASAP2600 surface analyzer from US. The average pore size of the sample was calculated according to the BJH method.

5. Compressive strength: measured according to HG/T 2782-2001.

6. XRD pattern: the sample was measured with X-ray diffractometer (D5005) from Siemens to obtain the XRD pattern. Specifically, 1g sample was obtained and measured with the X-ray diffractometer to obtain the XRD pattern, wherein the determination conditions included: Cu target, K$\alpha$ radiation, solid detector, tube voltage of 40kV, tube current of 40mA.

Raw materials:

Sodium metaaluminate, from Sinopharm; 13X, from Luoyang JALON Micro-nano New Materials Co. Ltd; NaY, from Luoyang JALON Micro-nano New Materials Co. Ltd; Sebania powder, from Sinopharm

Example 1

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=8. The reaction was operated at a temperature of 50 °C for 1 hour, to obtain a precipitate, which was washed and dried to obtain pseudo-boehmite. Tested as described above, the obtained pseudo-boehmite had a specific surface area of 330 m$^2$/g, a pore volume of 0.35 cm$^3$/g, an amount of weak acids of 0.4 mmol/g, and a pore size of 4.4 nm.

20 kg of the above-mentioned pseudo-boehmite was added into 20 kg of polyacrylic acid solution of 1.0% by mass. The obtained slurry was mixed evenly, dried at 100 °C for 8 hours, and then calcined at 550 °C for 6 hours to obtain alumina. Tested as described above, the obtained alumina had a specific surface area of 281m$^2$/g, a pore volume of 0.55 cm$^3$/g, an amount of weak acids of 0.02mmol/g, and a pore size of 7.8nm.

10 kg of 13X, 14 kg of the above-mentioned pseudo-boehmite, 6 kg of the alumina and 0.3 kg of sesbania powder were crushed, mixed evenly, after adding 5 kg of HNO$_3$ (a solution of 3 mass%), kneaded, extruded into stripe shape, and dried at 120 °C for 12 hours. The extruded stripe sample was calcined at 300 °C for 5 hours to obtain an adsorbent composition. Tested as described above, the adsorbent composition had a total amount of strong acids of 0.04 mmol/g, and a compressive strength of 80 N/cm.

The adsorption capacity for saturated brine was tested by: drying 10g of the adsorbent composition at 120 °C for 12 hours, getting its weight as WEIGHT 1, placing it into a dryer containing saturated brine for 24 hours, taking it out and getting its weigh as WEIGHT 2, and calculating the adsorption capacity for saturated brine of the adsorbent composition according to the following formula. The result was 18.9%:

$$\text{The adsorption capacity for saturated brine} = (\text{WEIGHT 2-WEIGHT 1}) / \text{WEIGHT 1} * 100\%$$

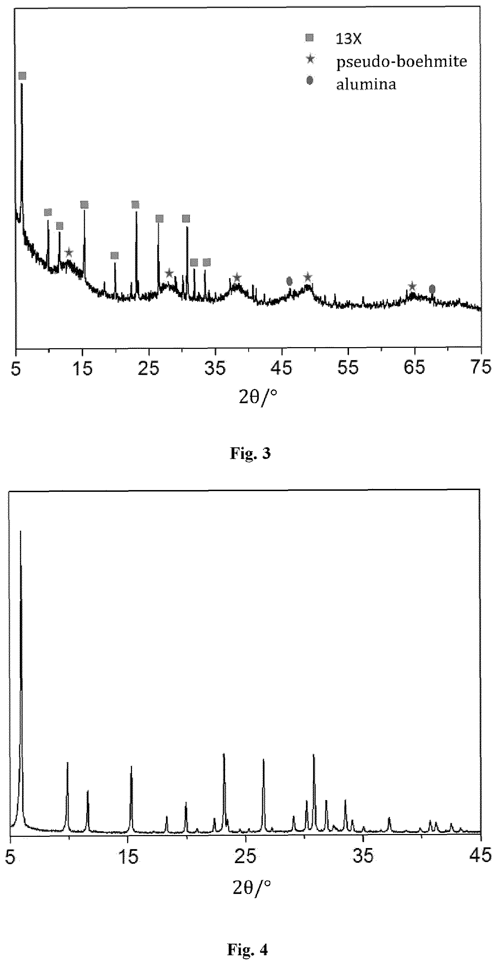

The XRD pattern of the adsorbent composition was tested as described above, and was showed in Fig. 3.

Example 2

500ml distilled water was added into a coprecipitation reactor (2000ml), to which 0.6mol/L sodium metaaluminate solution was added at a rate of 30ml/min, and 1.0mol/L nitric acid solution was added at the same time wherein the rate of adding nitric acid solution was controlled so that reaction was operated at pH=7. The reaction was operated at a temperature of 90 °C for 1.5 hours, to obtain a precipitate, which was washed and dried to obtain pseudo-boehmite. Tested as described above, the obtained pseudo-boehmite had a specific surface area of 380 m$^2$/g, a pore volume of 0.32 cm$^3$/g, an amount of weak acids of 0.45 mmol/g, and a pore size of 4.3 nm.

20 kg of the above-mentioned pseudo-boehmite was added into 10 kg of polyacrylic acid solution of 1.5% by mass. The obtained slurry was mixed evenly, dried at 150 °C for 6 hours, and then calcined at 600 °C for 5 hours to obtain alumina. Tested as described above, the obtained alumina had a specific surface area of 295m$^2$/g, a pore volume of 0.65 cm$^3$/g, an amount of weak acids of 0.03 mmol/g, and a pore size of 6.7nm.

10 kg of 13X, 10 kg of the above-mentioned pseudo-boehmite, 10 kg of the alumina and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 250 °C for 8 hours to obtain an adsorbent composition. Tested as described above, the adsorbent composition had a total amount of strong acids of 0.04 mmol/g, and a compressive strength of 64 N/cm.

The adsorption capacity for saturated brine was tested as described in Example 1. The result was 22.3%.

Example 3

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=6. The reaction was operated at a temperature of 50 °C for 1 hour, to obtain a precipitate, which was washed and dried to obtain pseudo-boehmite. Tested as described above, the obtained pseudo-boehmite had a specific surface area of 350 $m^2$/g, a pore volume of 0.37 $cm^3$/g, an amount of weak acids of 0.35mmol/g, and a pore size of 4.7 nm.

20 kg of the above-mentioned pseudo-boehmite was added into 30 kg of polyacrylic acid solution of 1.0% by mass. The obtained slurry was mixed evenly, dried at 100 °C for 8 hours, and then calcined at 550 °C for 6 hours to obtain alumina. Tested as described above, the obtained alumina had a specific surface area of 275$m^2$/g, a pore volume of 0.75 $cm^3$/g, an amount of weak acids of 0.03 mmol/g, and a pore size of 6.7nm.

6 kg of NaY, 18 kg of the above-mentioned pseudo-boehmite, 8 kg of the alumina and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 350 °C for 4 hours to obtain an adsorbent composition. Tested as described above, the adsorbent composition had a total amount of strong acids of 0.02 mmol/g, and a compressive strength of 85 N/cm.

The adsorption capacity for saturated brine was tested as described in Example 1. The result was 17.8%.

Example 4

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=8. The reaction was operated at a temperature of 50 °C for 1 hour, to obtain a precipitate, which was washed and dried to obtain pseudo-boehmite. Tested as described above, the obtained pseudo-boehmite had a specific surface area of 330 $m^2$/g, a pore volume of 0.35 $cm^3$/g, an amount of weak acids of 0.4mmol/g, and a pore size of 4.4 nm.

20 kg of the above-mentioned pseudo-boehmite was added into 10 kg of 1.0% by mass of polyacrylic acid solution. The obtained slurry was mixed evenly, dried at 100 °C for 8 hours, and then calcined at 500 °C for 6 hours to obtain alumina. Tested as described above, the obtained alumina had a specific surface area of 295$m^2$/g, a pore volume of 0.60 $cm^3$/g, an amount of weak acids of 0.03 mmol/g, and a pore size of 7.7nm.

3 kg of 13X, 21 kg of the above-mentioned pseudo-boehmite, 5 kg of the alumina and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 250 °C for 6 hours to obtain an adsorbent composition. Tested as described above, the adsorbent composition had a total amount of strong acids of 0.02 mmol/g, and a compressive strength of 80 N/cm.

The adsorption capacity for saturated brine was tested as described in Example 1. The result was 15.6%.

Example 5

10 kg of 13X, 14 kg of pseudo-boehmite (obtained from Jiangsu Sanji Industrial Co. Ltd., with a specific surface area of 312$m^2$/g and a pore volume of 0.34 $cm^3$/g), 6 kg of alumina (obtained from ShandongYunneng Catalytic Technology Co., Ltd., with a specific surface area of 297 $m^2$/g and a pore volume of 0.57 $cm^3$/g) and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 300 °C for 5 hours to obtain an adsorbent composition. Tested as described above, the adsorbent composition had a total amount of strong acids of 0.04 mmol/g, and a compressive strength of 40 N/cm.

The adsorption capacity for saturated brine was tested as described in Example 1. The result was 16.7%.

Comparative Example 1

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=9. The reaction was operated at a temperature of 50 °C for 1 hour, to obtain a precipitate, which was washed and dried to obtain pseudo-boehmite. Tested as described above, the obtained pseudo-boehmite had a specific surface area of 410 $m^2$/g, a pore volume of 0.84 $cm^3$/g, an amount of weak acids of 0.4mmol/g, and a pore size of 8.2 nm.

20 kg of the above-mentioned pseudo-boehmite was added into 20 kg of polyacrylic acid solution of 1.0% by mass. The obtained slurry was mixed evenly, dried at 100 °C for 8 hours, and then calcined at 400-600°C for 6 hours to obtain alumina. Tested as described above, the obtained alumina had a specific surface area of 350$m^2$/g, a pore volume of 0.65 $cm^3$/g, an amount of weak acids of 0.02mmol/g, and a pore size of 7.8 nm.

10 kg of 13X, 14 kg of the above-mentioned pseudo-boehmite, 6 kg of the alumina and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 500 °C for 5 hours to obtain an adsorbent composition. Tested as described above, the adsorbent composition had a total amount of strong acids of 0.1 mmol/g, and a compressive strength of 13 N/cm.

The adsorption capacity for saturated brine was tested as described in Example 1. The result was 21.3%.

Comparative Example 2

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=5. The reaction was operated at a temperature of 50 °C for 1 hour, to obtain a precipitate, which was washed and dried to obtain pseudo-boehmite. Tested as described above, the obtained pseudo-boehmite had a specific surface area of 250 $m^2$/g, a pore volume of 0.28 $cm^3$/g, an amount of weak acids of 0.28 mmol/g, and a pore size of 3.6 nm.

20 kg of the above-mentioned pseudo-boehmite was added into 20 kg of polyacrylic acid solution of 1.0% by mass. The obtained slurry was mixed evenly, dried at 100 °C for 8 hours, and then calcined at 550°C for 6 hours to obtain alumina. Tested as described above, the obtained alumina had a specific surface area of 217 $m^2$/g, a pore volume of 0.35 $cm^3$/g, an amount of weak acids of 0.01mmol/g, and a pore size of 5.4nm.

10 kg of 13X, 14 kg of the above-mentioned pseudo-boehmite, 6 kg of the alumina and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 450 °C for 5 hours to obtain an adsorbent composition. Tested as described above, the adsorbent composition had a total amount of strong acids of 0.07 mmol/g, and a compressive strength of 69 N/cm.

The adsorption capacity for saturated brine was tested as described in Example 1. The result was 10%.

Comparative Example 3

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=8. The reaction was operated at a temperature of 50 °C for 1 hour, to obtain a precipitate, which was washed and dried to obtain pseudo-boehmite. Tested as described above, the obtained pseudo-boehmite had a specific surface area of 330 $m^2$/g, a pore volume of 0.35 $cm^3$/g, an amount of weak acids of 0.4mmol/g, and a pore size of 4.4 nm.

10 kg of 13X, 20 kg of the above-mentioned pseudo-boehmite and 0.3 kg of sesbania powder were crushed, mixed evenly, after adding 5 kg of $HNO_3$ (a solution of 3 mass%), kneaded, extruded into stripe shape, and dried at 120 °C for 12 hours. The extruded stripe sample was calcined at 300 °C for 5 hours to obtain an adsorbent composition. Tested as described above, the adsorbent composition had a total amount of strong acids of 0.01mmol/g, and a compressive strength of 100 N/cm.

The adsorption capacity for saturated brine was tested as described in Example 1. The result was 18.6%.

Comparative Example 4

200ml distilled water was added into a coprecipitation reactor (2000ml), to which 1.0mol/L sodium metaaluminate solution was added at a rate of 10ml/min, and 1.0mol/L nitric acid solution was added at the same time wherein the rate of adding nitric acid solution was controlled so that reaction was operated at pH=8. The reaction was operated at a temperature of 50 °C for 1 hour, to obtain a precipitate, which was washed and dried to obtain pseudo-boehmite. Tested as described above, the obtained pseudo-boehmite had a specific surface area of 330 m$^2$/g, a pore volume of 0.35 cm$^3$/g, an amount of weak acids of 0.4mmol/g, and a pore size of 4.4 nm.

20 kg of the above-mentioned pseudo-boehmite was added into 20 kg of polyacrylic acid solution of 1.0% by mass. The obtained slurry was mixed evenly, dried at 100 °C for 8 hours, and then calcined at 400-600°C for 6 hours to obtain alumina. Tested as described above, the obtained alumina had a specific surface area of 281m$^2$/g, a pore volume of 0.55 cm$^3$/g, an amount of weak acids of 0.02mmol/g, and a pore size of 7.8nm.

10 kg of 13X, 20 kg of the alumina and 0.3 kg of sesbania powder were crushed, mixed evenly, after adding 5 kg of HNO$_3$ (a solution of 3 mass%), kneaded, extruded into stripe shape, and dried at 120 °C for 12 hours. The extruded stripe sample was calcined at 300 °C for 5 hours to obtain an adsorbent composition. Tested as described above, the adsorbent composition had a total amount of strong acids of 0.1 mmol/g, and a compressive strength of less than 10 N/cm.

The adsorption capacity for saturated brine was tested as described in Example 1. The result was 25%.

Comparative Example 5

30 kg of 13X and 0.3 kg of sesbania powder were crushed, mixed evenly, after adding 5 kg of HNO$_3$ (a solution of 3 mass%), kneaded, extruded into stripe shape, and dried at 120 °C for 12 hours. The extruded stripe sample was calcined at 300 °C for 5 hours to obtain an adsorbent composition. Tested as described above, the adsorbent composition had a total amount of strong acids of 0.15 mmol/g, and a compressive strength of less than 10 N/cm.

The adsorption capacity for saturated brine was tested as described in Example 1. The result was 22%.

The XRD pattern of the adsorbent composition was tested as described above, and was showed in Fig.4.

In the XRD pattern shown in Fig. 4, there were diffraction peaks at 2θ of 6.03°, 9.87°, 11.6°, 15.32°, 23.16°, 26.5° and 30.8°.

Work Example

This work example was to show the adsorption performances of the adsorbent composition on the adsorption of NH$_3$, H$_2$S and methanol under various conditions and the regeneration performances thereof.

Adsorption of NH$_3$ and H$_2$S: 5g of the adsorbent composition sample was incorporated into a fixed-bed reactor, into which nitrogen gas stream containing 1000ppm NH$_3$ and 1000ppm H$_2$S was injected at a flow rate of 100ml/min. The reactor was kept at room temperature (25 °C) and normal pressure (1 atm). The contents of H$_2$S and NH$_3$ in the sample were detected with Agilent SCD sulfur detector and NCD nitrogen detector respectively. When the content of NH$_3$ or H$_2$S in the outlet gas exceeded 1 ppm, breakthrough of the adsorbent was regarded as occurred.

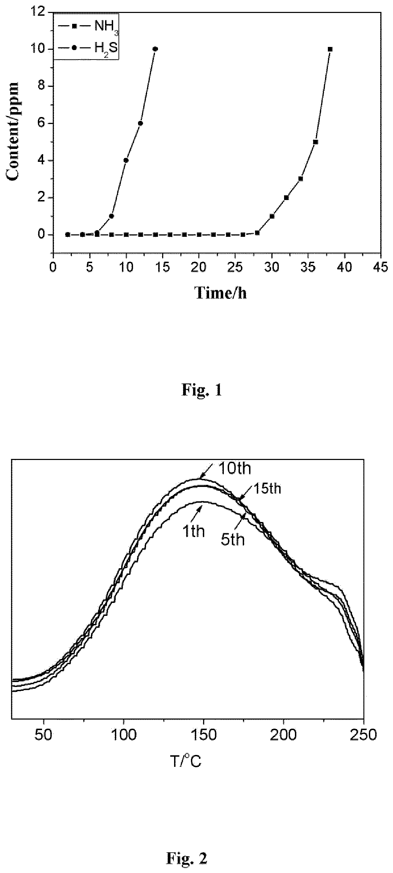

The adsorbent composition prepared in Example 1 was used as a sample and tested as described above. The content of H$_2$S or NH$_3$ at the outlet of the reactor was detected within a period of time, which was plotted as a curve of the content VS time, to get the breakthrough curve. The breakthrough curve of the adsorbent composition prepared in Example 1 was showed in Fig. 1.

The breakthrough volume was calculated based on the breakthrough time of the adsorbent obtained from the breakthrough curve by the following formula:

$$S = V \times t \times (C_{in} - C_{out}) \div 22.4 \div 1000 \times M \div mad \times 10^{-6};$$

V: the flow rate of gas stream (mL/min);
t: the breakthrough time (min);
mad: loading amount of the adsorbent (g);
M: the molar mass of H$_2$S or NH$_3$ (L/mol);
Cin: the content of H$_2$S or NH$_3$ at the inlet (ppm);
Cout: the content of H$_2$S or NH$_3$ at the outlet (ppm);

The adsorbent composition prepared in Example 1 had a breakthrough volume of 2.7% for NH$_3$, and of 1.4% for H$_2$S.

Adsorption of methanol: 20g of the adsorbent composition was incorporated into a fixed-bed reactor. Nitrogen gas stream was injected at a flow rate of 100ml/min to bubble through a vessel containing methanol solution at 30 °C, and then pass through the reactor which is at a temperature of 50 °C. The outlet gas was detected by gas chromatography to check whether there was any signal peak for methanol or not. If there was a signal peak for methanol, the adsorbent was in adsorption saturation. The weights of the adsorbent before and after adsorption saturation were detected with a balance to obtain the adsorption capacity for methanol of the adsorbent composition sample. The adsorption capacity for methanol = (the weight of the adsorbent composition after adsorption saturation - the weight of the adsorbent composition before adsorption saturation)/the weight of the adsorbent composition before adsorption saturation * 100%.

The adsorbent composition prepared in Example 1 was used as a sample and tested as described above. It had an adsorption capacity for methanol of 15%.

Effects of regeneration on the performances of the adsorbent composition: the saturated sample of the adsorbent was heated at 180 °C for 5 hours for regenerating to obtain the regenerated sample. The regenerated sample was tested as described above to obtain the adsorption capacity for saturated brine, the breakthrough volume for $NH_3$, the breakthrough volume for $H_2S$ and the adsorption capacity for methanol of the regenerated sample.

The adsorbent composition prepared in Example 1 was regenerated once as described above. The regenerated sample was tested to have an adsorption capacity for saturated brine of 18.9%, a breakthrough volume for $NH_3$ of 2.8%, a breakthrough volume for $H_2S$ of 1.3% and an adsorption capacity for methanol of 15%.

Adsorption of $NH_3$, $H_2S$ and methanol in the presence of ethylene: the above tests were repeated except that a nitrogen gas stream containing 1000 ppm $NH_3$ and 10% ethylene, a nitrogen gas stream containing 1000 ppm $H_2S$ and 10% ethylene, and a nitrogen gas stream containing 5% ethylene were passed through the methanol solution, respectively.

The adsorbent composition prepared in Example 1 was used in the above tests. The results were a breakthrough volume for $NH_3$ of 2.8%, a breakthrough volume for $H_2S$ of 1.3% and an adsorption capacity for methanol of 15%.

Reproducibility: 5g of the adsorbent composition sample was incorporated into a fixed-bed reactor, into which a nitrogen gas stream containing 1000ppm $NH_3$ and 5% propylene was injected at a flow rate 240ml/min. The reactor was kept at room temperature (25 °C) and normal pressure (1 atm). The content of $NH_3$ was detected with Agilent NCD nitrogen detector. When the content of $NH_3$ in the outlet gas exceeded 1 ppm, breakthrough of the adsorbent was regarded as occurred. The breakthrough curve of the adsorbent composition was obtained and the breakthrough volume for $NH_3$ was calculated. The used sample was heated at 180 °C for 5 hours for regenerating to obtain a regenerated sample. The above tests were repeated with the regenerated sample to obtain the breakthrough volume for $NH_3$ of the regenerated sample. The regeneration and tests were repeated for 15 times to obtain the breakthrough volume for $NH_3$ of each regenerated sample.

The adsorbent composition of Example 1 was used in the above tests. The fresh adsorbent composition had a breakthrough volume for $NH_3$ of 2.8%. The adsorbent composition regenerated for once had a breakthrough volume for $NH_3$ of 2.8%. The adsorbent composition regenerated for 15 times had a breakthrough volume for $NH_3$ of 2.7%.

Fig. 2 showed the $NH_3$-TPD curve of the adsorbent composition of Example 1 regenerated for several times. It could be seen from the figure that, for the adsorbent composition of Example 1, it was possible to remove $NH_3$ from the adsorbent at about 150 °C, which allowed online regeneration, and that the adsorbent performances were still maintained well after being regenerated for 15 times.

The adsorbent composition of Comparative Example 1 was used to repeat the above tests. The fresh adsorbent composition had a breakthrough volume for $NH_3$ of 2.9%. The adsorbent composition regenerated for once had a breakthrough volume for $NH_3$ of 2.7%. The adsorbent composition regenerated for 15 times had a breakthrough volume for $NH_3$ of 2.0%.

The adsorbent composition of Comparative Example 5 was used to repeat the above tests. The fresh adsorbent composition had a breakthrough volume for $NH_3$ of 3.2%. The adsorbent composition regenerated for once had a breakthrough volume for $NH_3$ of 1.0%. The adsorbent composition regenerated for 5 times had a breakthrough volume for $NH_3$ of 0.2%.

**Claims**

1. An adsorbent composition for removing polar compounds from low-carbon olefins, **characterized in that** the adsorbent composition comprises molecular sieves, hydrated alumina and alumina.

2. The adsorbent composition of claim 1, **characterized in that** the adsorbent composition comprises, by weight: a) 10-50 parts, preferably 20-40 parts of the molecular sieves; b) 20-55 parts, preferably 30-45 parts of the hydrated alumina, calculated as alumina; c) 5-35 parts, preferably 8-30 parts of the alumina.

3. The adsorbent composition of claim 1, **characterized in that** the adsorbent composition has a total amount of strong acids of less than 0.05mmol/g, preferably 0.01-0.04mmol/g

4. The adsorbent composition of claim 1, **characterized in that** the XRD pattern of the adsorbent composition involves diffraction peaks at 2θ of 6.03°, 13.47°, 15.32°, 28.01°, 37.96° and 49.28°, preferably involves diffraction peaks at 2θ of 6.03°, 13.47°, 15.32°, 28.01°, 23.16°, 37.96°, 46.34°, 49.28° and 66.8°, more preferably involves diffraction peaks at 2θ as outlined in the following table:

| 2θ |
| --- |
| 6.03 |
| 9.87 |
| 11.6 |
| 13.47 |
| 15.32 |
| 19.9 |
| 23.16 |
| 26.5 |
| 28.01 |
| 30.8 |
| 31.8 |
| 33.4 |
| 37.96 |
| 46.34 |
| 49.28 |
| 64.7 |
| 66.8 |

5. The adsorbent composition of claim 1, **characterized in that** the hydrated alumina comprises one or more of pseudo-boehmite, boehmite, gibbsite and bayerite, preferably is pseudo-boehmite, or
the hydrated alumina has a specific surface area of 300-400m$^2$/g, and a pore volume of 0.3-0.4 cm$^3$/g.

6. The adsorbent composition of claim 1, **characterized in that** the alumina has a specific surface area of 200-300m$^2$/g, and a pore volume of 0.5-0.8cm$^3$/g.

7. The adsorbent composition of claim 1, **characterized in that** the molecule sieves are one or two selected from the group consisting of X-type molecular sieves and Y-type molecular sieves, preferably one or two selected from the group consisting of 13X and NaY.

8. A method for preparing the adsorbent composition of any one of claims 1-7, **characterized in that** the method comprises the steps of:
subjecting molecular sieves, hydrated alumina and alumina to mixing, shaping, drying and calcining to obtain the adsorbent composition, wherein the calcining is operated at a temperature of lower than 400 °C.

9. The method of claim 8, **characterized in that** the hydrated alumina is pseudo-boehmite, preferably, the pseudo-boehmite prepared by coprecipitation reaction of an aqueous solution of water-soluble metaaluminates and a nitric acid solution, wherein the water-soluble metaaluminates are one or two of sodium metaaluminate and potassium metaaluminate, and the reaction is operated at pH=6-8.

10. The method of claim 8, **characterized in that** the calcining is operated at a temperature of 200-400 °C, preferably 250-350 °C for 2-10 hours, preferably 3-8 hours

11. A use of the adsorbent composition of any one of claims 1-7 or the adsorbent composition prepared by the method of any one of claims 8-10 in removing polar compounds from low-carbon olefins.

**Fig. 1**

**Fig. 2**

Fig. 3

Fig. 4

## INTERNATIONAL SEARCH REPORT

| | International application No. |
|---|---|
| | **PCT/CN2021/120556** |

**A.    CLASSIFICATION OF SUBJECT MATTER**

B01J 20/18(2006.01)i;  B01J 20/30(2006.01)i;  C07C 7/13(2006.01)i

According to International Patent Classification (IPC) or to both national classification and IPC

**B.    FIELDS SEARCHED**

Minimum documentation searched (classification system followed by classification symbols)

B01J20/-;  C07C7/-

Documentation searched other than minimum documentation to the extent that such documents are included in the fields searched

Electronic data base consulted during the international search (name of data base and, where practicable, search terms used)

CNABS, CNTXT, CNKI, VEN, web of science: 吸附, 脱除, 去除, 纯化, 净化, 沸石, 分子筛, 氧化铝, sorb+, absor+, remov+, desorp+, Al2O3, alumina, aluminum oxide, molecular sieve?, zeolite

**C.    DOCUMENTS CONSIDERED TO BE RELEVANT**

| Category* | Citation of document, with indication, where appropriate, of the relevant passages | Relevant to claim No. |
|---|---|---|
| X | CN 103611495 A (SHANGHAI LVQIANG NEW MATERIALS CO., LTD. et al.) 05 March 2014 (2014-03-05) <br> description, paragraphs 5-21, embodiment 10 | 1-11 |
| X | CN 1690169 A (CHINA PETROLEUM & CHEMICAL CORPORATION et al.) 02 November 2005 (2005-11-02) <br> embodiment 1 | 1-10 |
| A | CN 111097369 A (CHINA PETROLEUM & CHEMICAL CORPORATION et al.) 05 May 2020 (2020-05-05) <br> entire document | 1-11 |
| A | CN 106423114 A (DALIAN HAIXIN CHEMICAL INDUSTRIAL CO., LTD.) 22 February 2017 (2017-02-22) <br> entire document | 1-11 |
| A | CN 1359749 A (UOP L.L.C.) 24 July 2002 (2002-07-24) <br> entire document | 1-11 |
| A | US 2006283780 A1 (SUED CHEMIE INC.) 21 December 2006 (2006-12-21) <br> entire document | 1-11 |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 November 2021** | **30 December 2021** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2021/120556** |

**C. DOCUMENTS CONSIDERED TO BE RELEVANT**

| Category* | Citation of document, with indication, where appropriate, of the relevant passages | Relevant to claim No. |
|---|---|---|
| A | US 5284638 A (CORNING INC.) 08 February 1994 (1994-02-08)<br>entire document | 1-11 |

Form PCT/ISA/210 (second sheet) (January 2015)

**INTERNATIONAL SEARCH REPORT**
**Information on patent family members**

International application No.

**PCT/CN2021/120556**

| Patent document cited in search report | | | Publication date (day/month/year) | Patent family member(s) | | | Publication date (day/month/year) |
|---|---|---|---|---|---|---|---|
| CN | 103611495 | A | 05 March 2014 | CN | 103611495 | B | 28 October 2015 |
| CN | 1690169 | A | 02 November 2005 | CN | 1312255 | C | 25 April 2007 |
| CN | 111097369 | A | 05 May 2020 | None | | | |
| CN | 106423114 | A | 22 February 2017 | None | | | |
| CN | 1359749 | A | 24 July 2002 | CN | 1230247 | C | 07 December 2005 |
| | | | | IT | MI20012575 | A1 | 06 June 2003 |
| | | | | US | 2002147377 | A1 | 10 October 2002 |
| | | | | US | 6632766 | B2 | 14 October 2003 |
| | | | | CA | 2364052 | A1 | 08 June 2002 |
| | | | | CA | 2364052 | C | 31 August 2010 |
| | | | | DE | 10159525 | A1 | 19 February 2004 |
| | | | | DE | 10159525 | B4 | 27 June 2013 |
| | | | | FR | 2817772 | A1 | 14 June 2002 |
| | | | | FR | 2817772 | B1 | 27 January 2006 |
| | | | | JP | 2002253959 | A | 10 September 2002 |
| | | | | JP | 4185279 | B2 | 26 November 2008 |
| US | 2006283780 | A1 | 21 December 2006 | WO | 2007021554 | A2 | 22 February 2007 |
| | | | | WO | 2007021554 | A3 | 03 May 2007 |
| US | 5284638 | A | 08 February 1994 | EP | 0583594 | A1 | 23 February 1994 |
| | | | | EP | 0583594 | B1 | 12 June 1996 |
| | | | | AT | 139140 | T | 15 June 1996 |
| | | | | BR | 9303236 | A | 08 March 1994 |
| | | | | AU | 4428893 | A | 10 February 1994 |
| | | | | CA | 2099693 | A1 | 06 February 1994 |
| | | | | DE | 69303124 | D1 | 18 July 1996 |
| | | | | DE | 69303124 | T2 | 16 January 1997 |
| | | | | KR | 940003597 | A | 12 March 1994 |
| | | | | MX | 9304738 | A | 28 February 1994 |
| | | | | TW | 280779 | B | 11 July 1996 |
| | | | | JP | H06170145 | A | 21 June 1994 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1461290 A
- CN 103418164 B
- CN 107970781 A